# EUROPEAN PATENT SPECIFICATION

(11) **EP 4 454 600 B1**
(45) Date of publication and mention of the grant of the patent: **12.03.2025**
(21) Application number: 23178047.9
(22) Date of filing: 26.07.2022
(51) Int. Cl.: A61C 8/00, A61F 2/86

(54) **IMPLANT**
IMPLANTAT
IMPLANT

(30) Priority: 06.06.2022 TW 111120886
(43) Date of publication of application: 30.10.2024
(62) Divisional of application: 22187019.9
(73) Proprietor: Sentronic International Corp., New Taipei City (TW)
(72) Inventor: TSAI, Tung-Kuo, New Taipei City (TW)
(74) Representative: Melchior, Robin

(56) References cited:
- WO-A1-2020/222035
- KR-B1- 102 161 197
- US-A- 4 729 766
- US-A1- 2008 145 400

## Description

### BACKGROUND

### 1. Field of the Invention

The present invention relates to an implant that is configured to put into a human body, especially to an implant with surface treatment.

### 2. Description of the Prior Arts

In many surgeries, artificial objects may be put into human bodies, and these artificial objects are so-called implants. One of the common implants is an artificial tooth root for the dental implant surgery, and another one is a stent for cardiac catheterization. The artificial tooth root is configured to be inserted into the gum, so the gum should be drilled and thus form a hole for the artificial tooth root. After a period of time, the bone of the gum may grow and thus the artificial tooth root can be secured on the gum. However, the growth speed of the bone is very slow, which causes inconvenience to the patient after the surgery. Thus, how to improve the growth speed of the bone is an issue of the dental implant surgery. On the contrary, after the cardiac catheterization, if new blood vessels are developed (i.e. angiogenesis), the implanted blood vessel is prone to blockage again. Thus, how to prevent angiogenesis is an issue of the cardiac catheterization. US 2008/145400 A1 describes an endoprosthesis which includes a tubular matrix with pores.

### SUMMARY OF THE INVENTION

To overcome the shortcomings, the present invention provides an implant to mitigate or obviate the aforementioned problems.

The main objective of the present invention is to provide an implant that is capable of absorbing more medicine, such that the medicine can assist the implanted tissue of the human body in recovery, or prevent the implanted tissue from development.

The implant has an outer surface and a plurality of cavities. The outer surface forms a plurality of openings. The cavities communicates with the openings respectively.

The implant which is a stent has a plurality of wire structures and a plurality of tube structures. The tube structures are formed on each one of the wire structures. Each one of the tube structures forms a cavity, one end of each one of the tube structures is connected to the wire structure, and another end forms an opening communicating with the cavity.

Therefore, because the outer surface of the implant of the present invention forms multiple openings and the openings communicate with the cavities in the implant, the medicine may flow into the cavities after applied on the implant. The latitudinal section of each cavity is larger than the corresponding opening, so after flowing into the cavities, the medicine would not easily flow out, which allows the implant to absorb more medicine.

Other objectives, advantages and novel features of the invention will become more apparent from the following detailed description when taken in conjunction with the accompanying drawings.

### BRIEF DESCRIPTION OF THE DRAWINGS

Fig. 1 is a perspective view of an implant in accordance with a first embodiment of an implant not according to the present invention;
Fig. 2 is a sectional view of the implant of the first embodiment in Fig. 1;
Fig. 3 is a sectional view of the implant in another configuration of the first embodiment in Fig. 1;
Fig. 4 is a sectional view of the implant in another configuration of the first embodiment in Fig. 1;
Fig. 5 is a sectional view of the implant in another configuration of the first embodiment in Fig. 1;
Fig. 6 is a perspective view of an implant in accordance with a second embodiment of an implant not according to the present invention;
Fig. 7 is a sectional view of the implant of the second embodiment in Fig. 6;
Fig. 8 is a perspective view of one of wire structures of the implant in another configuration of the second embodiment in Fig. 6, which configuration is according to the invention;
Fig. 9 is a sectional view of one of the wire structures of the implant in said another configuration of the second embodiment in Fig. 8;
Fig. 10 is a sectional view of another type of the implant in said another configuration of the second embodiment in Fig. 8;
Fig. 11 is a sectional view of another type of the implant in said another configuration of the second embodiment in Fig. 8;
Fig. 12 is a sectional view of another type of the implant in said another configuration of the second embodiment in Fig. 8; and
Fig. 13 is a sectional view of another type of the implant in said another configuration of the second embodiment in Fig. 8.

### DETAILED DESCRIPTION OF THE INVENTION

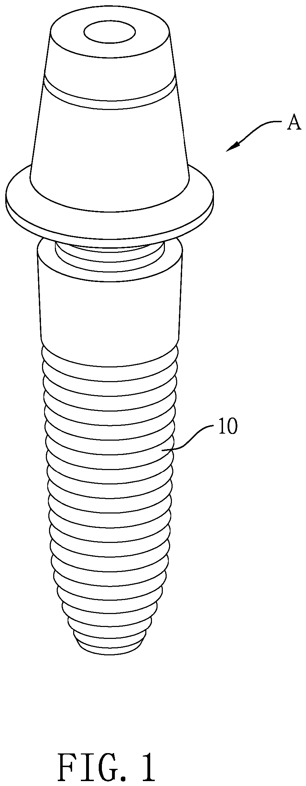

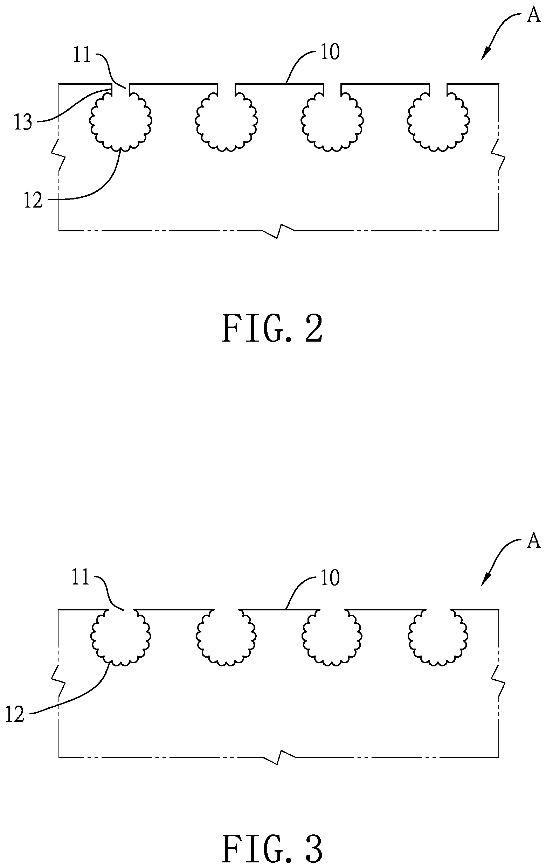

With reference to Fig.1 and Fig. 2, an implant not in accordance with the present invention is provided. In a first embodiment, the implant is an artificial tooth root A for the dental implant surgery and comprises an outer surface 10 and a plurality of cavities 12. The implant selectively further comprises a plurality of passages 13.

The outer surface 10 forms a plurality of openings 11. The cavities 12 respectively communicate with the openings 11. In this embodiment, each one of the cavities 12 communicates with the corresponding opening 11 via a respective one of the passages 13. Precisely, one end of each passage 13 communicates with one of the openings 11 and another end of said passage 13 communicates with one of the cavities 12. In this embodiment, each opening 11 communicates with one passage 13 and one cavity 12; in another embodiment, one opening 11 may communicate with one cavity 12 only. Fig. 2 shows the arrangement of the cavities 12. In Fig. 2, the openings 11 and the cavities 12 are arranged regularly on the outer surface 10; in another embodiment, the openings 11 and the cavities 12 may be arranged irregularly as shown in Fig. 3. In another configuration of this embodiment, the implant may not comprise any passage, such that the cavities 12 communicate with the openings 11 directly.

The longitudinal section of each one of the cavities 12 is perpendicular to the outer surface 10 and may be round, elliptical, oval, or triangular. The latitudinal section of each one of the cavities 12 is parallel with the outer surface 10, and the latitudinal section thereof is changed according to the distance from the outer surface. The maximum area of the latitudinal section is larger than the sectional area of the communicating opening 11. Therefore, the cavities 12 have more spaces to contain medicine. Further, the surface of each one of the cavities 12 may be smooth, or comprise irregular concave and convex structures.

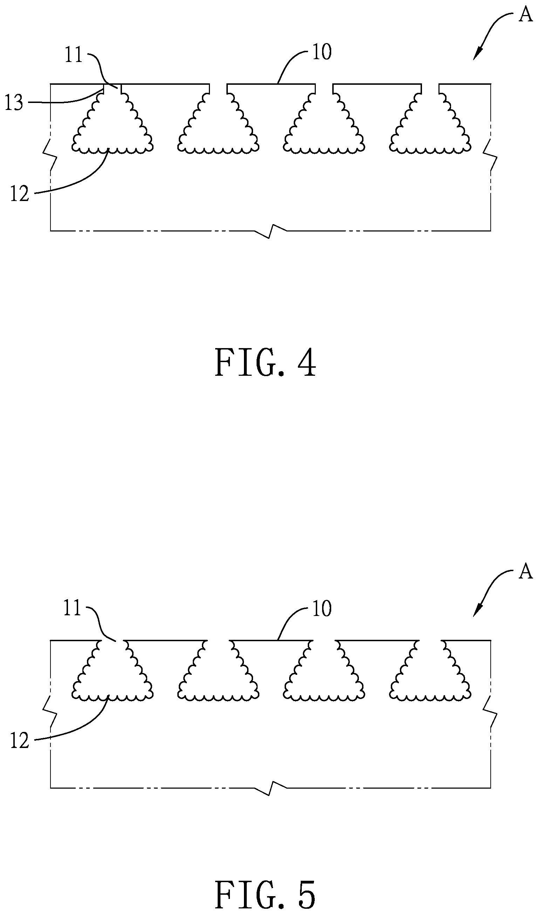

In the configurations as shown in Fig. 4 and Fig. 5, the latitudinal section of each one of the cavities 12 is as larger as farer from the outer surface 10. In other words, in these configurations, the longitudinal sections of the cavities 12 may be triangular.

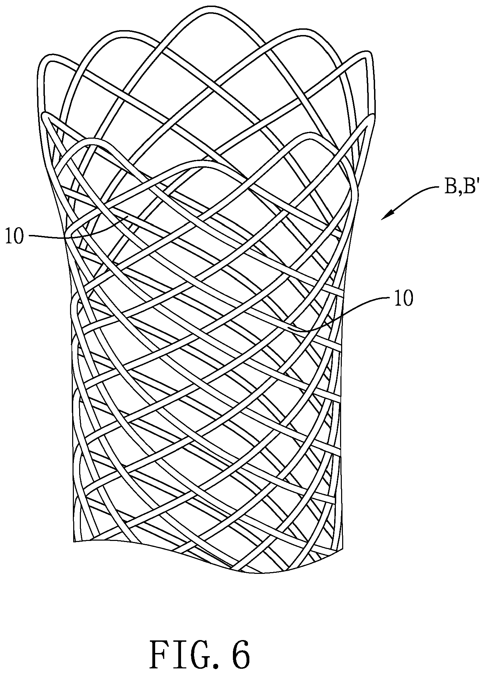

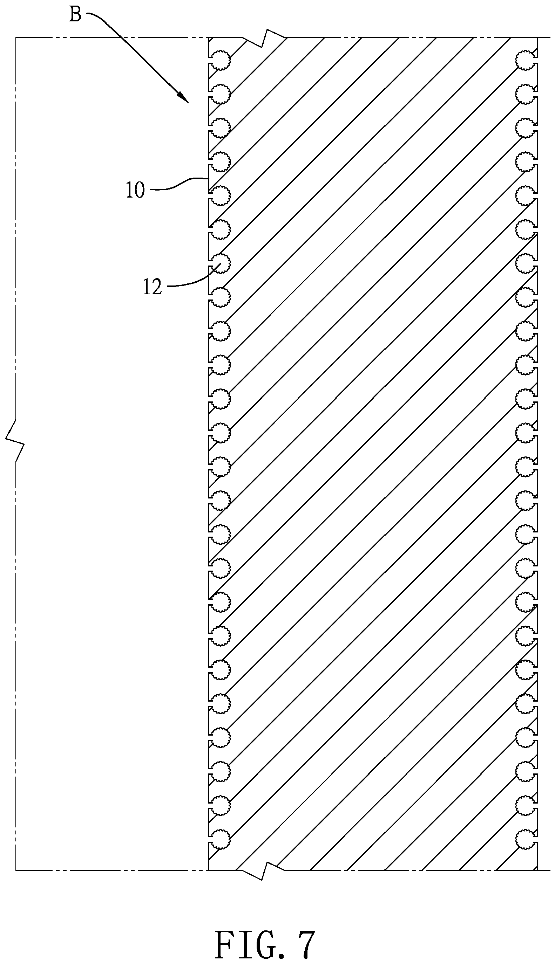

Then please refer to Fig. 6 and Fig. 7. In a second embodiment of an implant not according to the present invention, the implant is a stent B for cardiac catheterization. The stent B is roughly in the shape of a tube, so the outer surface 10 of the stent B includes an outward cylinder surface and an inward cylinder surface. In this embodiment, both of the outward cylinder surface and the inward cylinder surface form the openings 11 and the cavities 12; however, in another embodiment, the openings 11 and the cavities 12 may only be formed on the outward cylinder surface.

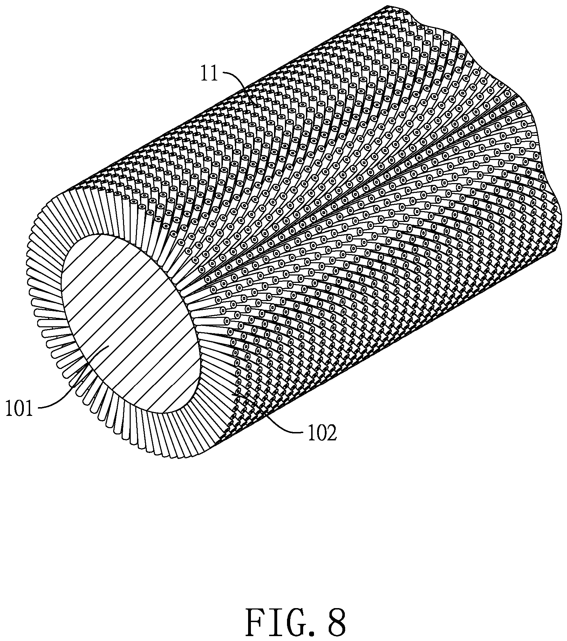

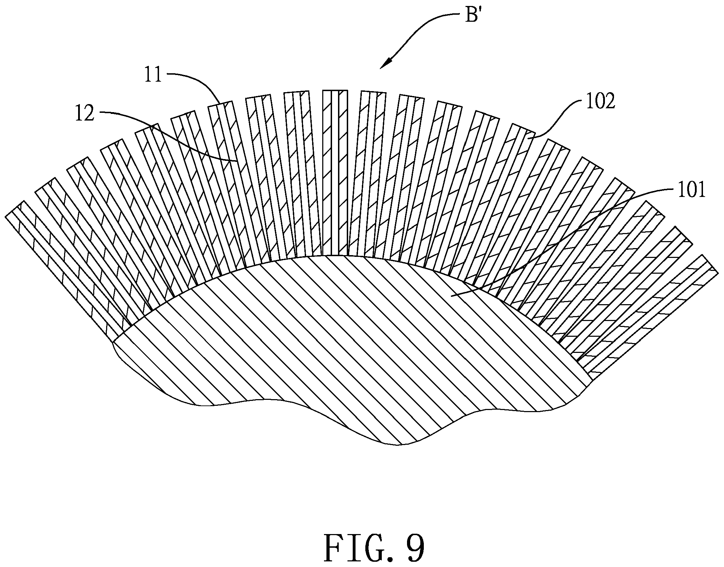

Then please refer to Fig. 8 and Fig. 9, which shows another configuration of the stent B' in the second embodiment. Fig. 8 shows one of the wires that forms the mesh tube (i.e. the stent B') and Fig. 9 shows the sectional view of the wire. Each one of the wire structures 101 of the stent B' forms a plurality of tube structures 102 located thereon and arranged densely. For example, ends of the tube structures 102 that are connected to the wire structure 101 are adjacent to each other. Each one of the tube structures 102 forms a cavity 12 therein. One end of each tube structure 102 is connected to the wire structure 101 and another end forms the aforementioned opening 11. The opening 11 communicates with the cavity 12. Because the tube structures 102 are arranged densely, end surfaces of the ends of the tube structures 102 that are away from the wire structure 101 form the outer surface of the stent B' together.

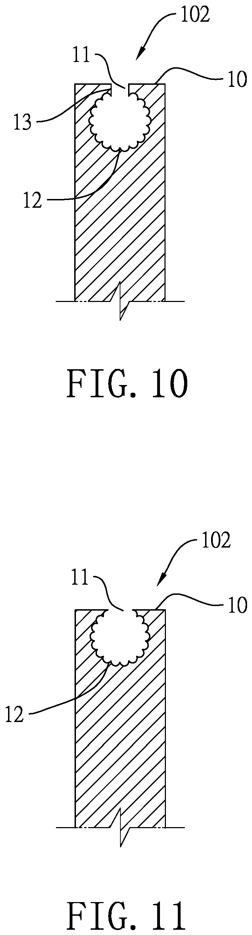

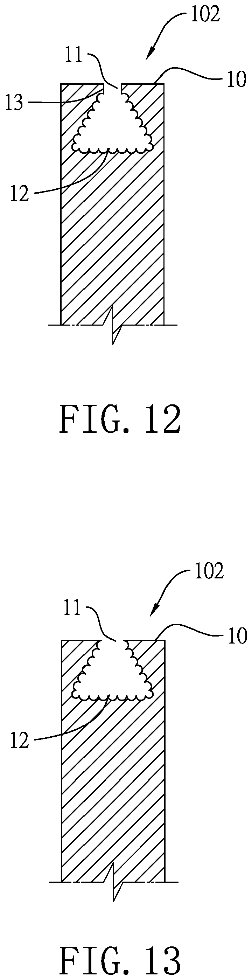

As shown in Figs. 10 to 13, the cavity of each end of the tube structures 102 may be round, elliptical, oval, or triangular. The latitudinal section of each one of the cavities 12 is parallel with the outer surface 10, and the latitudinal section thereof is changed according to the distance from the outer surface. The maximum area of the latitudinal section is larger than the sectional area of the communicating opening 11. Further, the surface of each one of the cavities 12 may be smooth, or comprise irregular concave and convex structures.

In some embodiments, each one of the cavities 12 communicates with the corresponding opening 11 via a respective one of the passages 13. Precisely, one end of each passage 13 communicates with one of the openings 11 and another end of said passage 13 communicates with one of the cavities 12. In some embodiments, each opening 11 communicates with one passage 13 and one cavity 12; in another embodiment, one opening 11 may communicate with one cavity 12 only.

With the aforesaid structure, the outer surface of the implant forms multiple openings 11 communicating with the cavities 12, and therefore the medicine can flow into the cavities 12 after the medicine is applied on the surface of the implant. Because the maximum of the latitudinal section of each cavity 12 is larger than the corresponding opening 11, the medicine may not flow out after flowing into the cavity 12, which allows the implant to absorb more medicine. If the implant is an artificial tooth root A, the medicine may include ingredients for enhancing the gum bone growth. Therefore, the implant of the present invention can absorb more medicine that enhances bone growth, which shortens the period for fixing the artificial tooth root A on the gum. On the other hand, if the implant is a stent B, the medicine may include ingredients for preventing angiogenesis. Therefore, the implant of the present invention can absorb more medicine that prevents angiogenesis, which decreases vascular blockage possibility.

## Claims

1. An implant **characterized in that** the implant comprises:
a plurality of wire structures (101); and
a plurality of tube structures (102) formed on each one of the wire structures (101); each one of the tube structures (102) forming a cavity (12), one end of each one of the tube structures (102) connected to the wire structure (101), and another end forming an opening (11) communicating with the cavity (12); the ends of the tube structures (102) with the opening (11) forming an outer surface,
wherein the implant is a stent (B').

2. The implant as claimed in claim 1, wherein a maximum area of a latitudinal section of each one of the cavities (12) is larger than a sectional area of the corresponding opening (11) and the latitudinal sections thereof are parallel with the outer surface (10).

3. The implant as claimed in claim 1 further comprising a plurality of passages (13), one end of each one of the passages (13) communicating with a respective one of the openings (11), and another end of each one of the passages (13) communicating with a respective one of the cavities (12).

4. The implant as claimed in any one of claims 1 to 3, wherein a longitudinal section of each one of the cavities (12) is round, elliptical, oval, or triangular; the longitudinal sections of the cavities (12) are perpendicular to the outer surface (10).

5. The implant as claimed in any one of claims 1 to 4, wherein a latitudinal section of each one of the cavities (12) is as larger as farer from the outer surface (10); the latitudinal sections of the cavities (12) are parallel with the outer surface (10).

## Patentansprüche

1. Implantat, **dadurch gekennzeichnet, dass** das Implantat umfasst:
eine Vielzahl von Drahtstrukturen (101); und
eine Vielzahl von Rohrstrukturen (102), die auf jeder der Drahtstrukturen (101) ausgebildet sind;
wobei jede der Rohrstrukturen (102) einen Hohlraum (12) bildet,
wobei ein Ende jeder der Rohrstrukturen (102) mit der Drahtstruktur (101) verbunden ist und ein anderes Ende eine Öffnung (11) bildet, die mit dem Hohlraum (12) in Verbindung steht;
wobei die Enden der Rohrstrukturen (102) mit der Öffnung (11) eine Außenfläche bilden, und
wobei das Implantat ein Stent (B') ist.

2. Implantat nach Anspruch 1, bei dem eine maximale Fläche eines Breitenabschnitts jedes der Hohlräume (12) größer als eine Querschnittsfläche der entsprechenden Öffnung (11) ist und die Breitenabschnitte davon parallel zu der Außenfläche (10) sind.

3. Implantat nach Anspruch 1, ferner umfassend eine Vielzahl von Durchgängen (13), wobei ein Ende jedes der Durchgänge (13) mit einer jeweiligen der Öffnungen (11) in Verbindung steht und ein anderes Ende jedes der Durchgänge (13) mit einem jeweiligen der Hohlräume (12) in Verbindung steht.

4. Implantat nach einem der Ansprüche 1 bis 3, bei dem ein Längsschnitt jedes der Hohlräume (12) rund, elliptisch, oval oder dreieckig ist; wobei die Längsschnitte der Hohlräume (12) senkrecht zu der Außenfläche (10) sind.

5. Implantat nach einem der Ansprüche 1 bis 4, bei dem ein Breitenabschnitt jedes der Hohlräume (12) so größer wie weiter von der Außenfläche (10) entfernt ist; wobei die Breitenabschnitte der Hohlräume (12) parallel zu der Außenfläche (10) sind.

## Revendications

1. Implant, **caractérisé en ce qu'**il comprend:
une pluralité de structures filaires (101); et
une pluralité de structures tubulaires (102) formées sur chacune des structures filaires (101);
chacune des structures tubulaires (102) formant une cavité (12),
une extrémité de chacune des structures tubulaires (102) étant connectée aux structures filaires (101), et une autre extrémité formant une ouverture (11) en communication avec la cavité (12);
les extrémités des structures tubulaires (102) comportant ladite ouverture (11) formant une surface extérieure,
dans lequel l'implant est une endoprothèse (B').

2. Implant selon la revendication 1, dans lequel une surface maximale d'une section latitudinale de chacune des cavités (12) est supérieure à une surface de section de l'ouverture correspondante (11), et en ce que les sections latitudinales de celles-ci sont parallèles à la surface extérieure (10).

3. Implant selon la revendication 1, comprenant en outre une pluralité de passages (13), une extrémité de chacun des passages (13) étant en communication avec une ouverture (11) correspondante, et une autre extrémité de chacun des passages (13) étant en communication avec une cavité (12) correspondante.

4. Implant selon l'une quelconque des revendications 1 à 3, dans lequel une section longitudinale de chacune des cavités (12) est ronde, elliptique, ovale ou triangulaire ; les sections longitudinales des cavités (12) étant perpendiculaires à la surface extérieure (10).

5. Implant selon l'une quelconque des revendications 1 à 4, dans lequel une section latitudinale de chacune des cavités (12) est d'autant plus large qu'elle est éloignée de la surface extérieure (10) ; les sections latitudinales des cavités (12) étant parallèles à la surface extérieure (10).
